# EUROPEAN PATENT APPLICATION

(11) **EP 1 221 320 A2**
(43) Date of publication of application: **10.07.2002**
(21) Application number: 01130664.4
(22) Date of filing: 21.12.2001
(51) Int. Cl.: A61M 5/14, A61M 5/162, A61M 5/165, A61M 5/168, A61M 39/24

(54) **Method of composing infusion line**

(30) Priority: 26.12.2000 JP 2000394867
(71) Applicant: JMS Co., Ltd., Hiroshima-shi, Hiroshima 730-8652 (JP)
(72) Inventor: Sawa, Kenji, Hiroshima-shi, Hiroshima 736-0086 (JP)
(74) Representative: Schwarzensteiner, Marie-Luise

(57) **Abstract**

Components for composing an infusion line are classified into unit groups including a spike unit (1), a main tube unit (2), an injection device unit (3), an infusion filter unit (4), and a one-way valve unit (5). Plural kinds of units are provided for each unit group, each unit being formed of one or more components. A combination of the unit groups is determined by allowing the spike unit and the main tube unit to be indispensable and selecting at least one of the unit groups from the remaining unit groups. An arrangement of the selected unit groups is determined, with the spike unit being positioned at one end. At least one kind of the units is selected from each of the unit group based on the combination. The selected units are combined, with the end on the distal side relative to the spike unit being composed of a connector used for a connection to a member for access to a patient.

## Description

The present invention generally relates to a method of composing an infusion line or transfusion line used as a conduit line for connecting a supply source of a medical fluid to a member for access to a patient such as a catheter, an injection needle and so on. In particular, the present invention relates to a method of composing the infusion line capable of performing simply the operation of combining various components to compose the infusion line. In the present invention, the term "infusion line" is used so as to have a meaning that includes "transfusion line".

Various kinds of infusion lines are used. For example, in operating rooms or intensive care units, an infusion line is used as a part of an arterial pressure measuring line for monitoring a blood pressure, or in some cases, a part of an infusion line is used as a blood collecting line. Methods of using such infusion lines differ from each other depending on hospitals, doctors or other medical staffs, and various kind of application. Therefore a large number of specifications of infusion lines are employed actually.

The specific infusion lines as mentioned above are so-called custom-made articles and are not used for general purposes, so that components thereof cannot be exchanged between infusion lines with different specifications. Accordingly, infusion tubes, functional elements, and connecting tools are made into many different shapes, sizes, or functions, to meet various different demands on clinical sites, and various kinds of infusion lines are manufactured by using different combinations thereof.

Thus conventional infusion lines mostly are custom-made, so that a manufacturing cost is high. Further, since it is necessary to design respective components independently in each specification, there is a problem that it takes a lot of time from order until manufacture and delivery of the infusion lines. Besides, the most important problem is that the knowledge of how to compose and use the infusion lines is limited to the range of individual experiences of persons in charge such as doctors and other medical staffs, and is not systematized. Therefore, compositions of the infusion lines are modified, and connections of the infusion lines to various apparatuses or ways of using the infusion lines are determined, according to subjective decisions of the persons in charge. Since the infusion lines are used for human bodies directly, there is a safety problem on the above-mentioned assembling of infusion lines.

As a method of solving the above-described problems, JP63(1988)-95063A discloses a system in which a circuit for an extracorporeal circulation process of a body fluid such as hemodialysis is configured according to the following steps: Several kinds of block configurations are made by unifying two or more functional parts into one block configuration; and some selected ones of the block configurations are combined to compose an infusion line. That system makes it possible to standardize components and manufacture the circuit easily to some extent. However, since the components incorporated into the block configurations are fixed, a circuit system has less flexibility in configuration and cannot meet diversified demands sufficiently.

Therefore, it is an object of the present invention to provide a method of composing an infusion line that enables an operation of composing an infusion line to be performed simply by selecting standardized units, on the basis of collections of users' various techniques and demands with respect to the composition of the infusion line and systematization of the infusion line as a combination of the standardized units.

A method of composing an infusion line according to the present invention includes the following steps:

Components for forming the infusion line are classified into unit groups including a spike unit, a main tube unit, an injection device unit, an infusion filter unit, and a one-way valve unit. Plural kinds of units are provided for each unit group according to different specifications, each unit being formed of one or more components combined based on a function of the respective unit. A combination of the unit groups is determined by allowing the spike unit and the main tube unit to be indispensable and selecting at least one of the unit groups from the remaining unit groups. An arrangement of the selected unit groups is determined, with the spike unit being positioned at one end thereof. At least one kind of the units is selected from each of the unit group based on the determined combination. The selected units are combined according to the determined arrangement to compose the infusion line, with the end on the distal side relative to the spike unit being composed of a connector used for a connection to a member for access to a patient.

According to the above method, it is possible to systematize the infusion line reasonably as the combination of a plurality of units based on the basic functions of respective elements and to perform simply the operation of composing an infusion line that meets users' demands by selecting the standardized units. Since each unit has compatibility and is made uniform, reduction in cost due to simplification of a production system becomes possible.

In the method described above, the unit groups may further include an extension tube unit, and when the extension tube unit is selected as one of the unit groups composing the inision line, the arrangement of the selected unit groups is determined with the extension tube unit being positioned at the end on the distal side relative to the spike unit. The combination of the unit groups may be determined with the extension tube unit being included in the indispensable units.

In the method described above, the spike unit may be positioned at an end portion on a medical fluid side of the infusion line and may have a function of connecting the infusion line to a supply source of a medical fluid via a spike. The main tube unit may be connected to the spike unit and may have a function of being used for adjusting a length between the spike unit and another unit positioned on a patient side. The injection device unit may be positioned between the main tube unit and the extension tube unit and may have a function of being used for injecting the medical fluid or collecting blood. The infusion filter unit may be positioned between the main tube unit and the extension tube unit and may have a function of being used for removing foreign substances (particles). The one-way valve unit may be positioned between the main tube unit and the extension tube unit and adjacent to the injection device unit and may have a function of preventing a back flow of an infusion fluid. The extension tube unit may be positioned at an end portion on the patient side and may have a function of allowing the infusion line to be held at hand of the patient easily.

Further, in the method described above, the spike unit may include a spike and a drip chamber as the components for forming the unit group. The main tube unit may include a tube, a damp for controlling flow rate, and a connector. The injection device unit may include a connector and at least one of an injection port and a three-way stop cock. The infusion filter unit may include an I-V filter, a tube for connection, and a connector. The one-way valve unit may include a one-way valve and a connector. The extension tube unit may include a tube and a connector.

The present invention will be more fully described hereinafter, with references being made to the accompanying drawings.

FIG. 1 is a plan view showing an infusion line, with sections of respective units being indicated according to a first embodiment of the present invention.

FIGs. 2A to 2F are views showing examples of a spike unit in the infusion line of FIG. 1.

FIGs 3Ato 3G are views showing examples of a main tube unit in the infusion line of FIG. 1.

FIGs. 4A to 4F are views showing examples of an injection device unit in the infusion line of FIG. 1.

FIGs. 5A to 5B are views showing examples of a one-way valve unit in the infusion line of FIG. 1.

FIGs. 6A to 6C are views showing examples of an infusion filter unit in the infusion line of FIG. 1.

FIGs. 7A to 7G are views showing examples of an extension tube unit in the infusion line of FIG. 1.

FIG. 8 is a plan view showing an infusion line, with sections of respective units being indicated according to a second embodiment of the present invention.

### [First Embodiment]

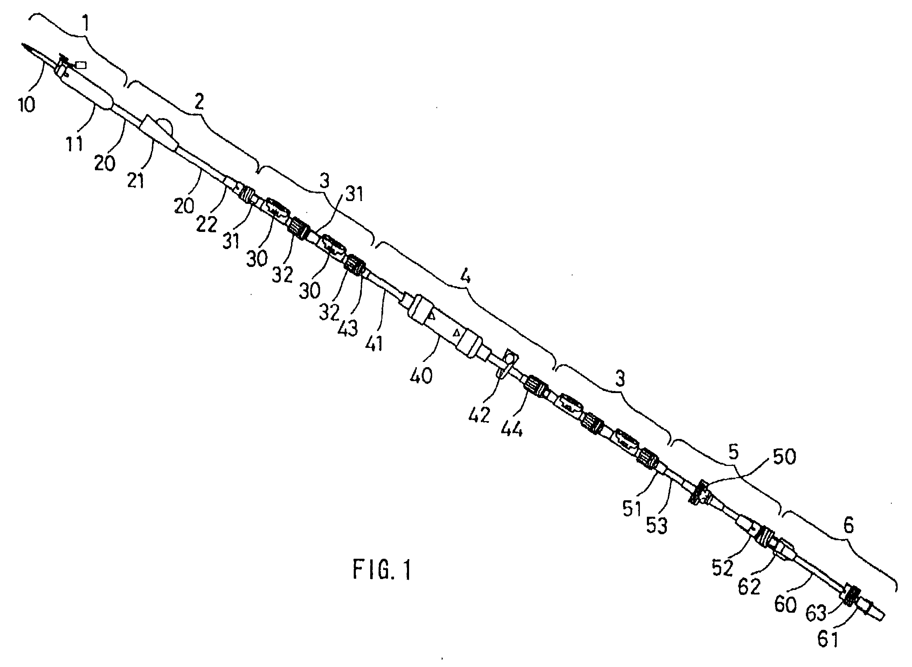

FIG. 1 shows a composition of an infusion line in a first embodiment according to the present invention, in which sections of respective units are indicated in order to describe a method of composing the infusion line. According to the method, components for forming the infusion line are classified into six unit groups and combined with each other so as to form several kinds of standardized units in each unit group, and a plurality of units selected from the unit groups are combined to compose the standardized infusion line. As shown in FIG. 1, the infusion line is divided into the unit groups such as a group of a spike unit 1, a group of a main tube unit 2, a group of an injection device unit 3, a group of an infusion filter unit 4, a group of a one-way valve unit 5, and a group of an extension tube unit 6.

When an infusion line for practical use is composed based on the above-described unit groups, some of the unit groups are selected according to a function of an infusion line, respective units are selected appropriately from the selected unit groups, and the selected units are combined. At this time, indispensable units are selected from each of the spike unit 1, the main tube unit 2, and the extension tube unit 6. And at least one kind of unit is selected from the injection device unit 3, the infusion filter unit 4, and the one-way valve unit 5. In an arrangement of the unit groups, the spike unit 1 is positioned at one end of the infusion line and the extension tube unit 6 is positioned at the other end The other units are, for example, interposed between the main tube unit 2 and the extension tube unit 6. Aplurality of the same kind of units may be selected from each of the selected unit group.

The basic functions of the respective units typically are realized by combining a plurality of components. Thus the term "unit" means the combination of components for realizing the function of each unit. However, one unit may be formed of one component. The term "spike unit 1", or "main tube unit 2", etc., means the names of the respective unit groups. The functions of units included in each unit group and the standard configurations of those units, i.e., the combination of components are as follows.

The spike unit 1 includes a spike 10 and a drip chamber 11 as the components. The spike unit 1 is positioned at an end portion on a medical fluid side (the distal side relative to a patient) and has a function of connecting the infusion line to a supply source of the medical fluid via the spike 10.

The main tube unit 2 includes a tube 20, a clamp 21 for controlling a flow rate, and a connector 22 as the components. The main tube unit 2 is connected to the spike unit 1 and is used for adjusting a length between the spike unit 1 and another unit positioned on a patient side.

The injection device unit 3 includes an injection port 30 and connectors 31 and 32 as the components. In some cases, in place of the injection port 30, a three-way stop cock (not shown) is used for the injection device unit 3. The injection device unit 3 is positioned between the main tube unit 2 and the extension tube unit 6. As shown in FIG. 1, in some cases, a plurality of injection device units 3 are used. The injection device unit 3 is used for injecting the medical fluid or collecting blood from the injection port or the three-way stop cock.

The infusion filter unit 4 includes a I-V filter 40 of a hollow fiber type, a connection tube 41, a clamp 42, and connectors 43 and 44 as the components. Although the clamp 42 is not indispensable for this unit, it is preferable that the unit includes the clamp 42 in order to fill up a fluid rapidly into a housing of the filter or remove air bubble easily. The infusion filter unit 4 is positioned between the main tube unit 2 and the extension tube unit 6 and is used for removing foreign substances. The foreign substances include junk of gums or corks, air bubbles, bacteria, virus, or the like.

The one-way valve unit 5 includes a one-way valve 50 and connectors 51 and 52 as the components. If required, the one-way valve unit 5 includes a tube 53. This unit is used for preventing a back flow of an infusion fluid and is positioned adjacent to the injection device unit 3 whereby the infusion fluid is prevented from flowing back in the line when a fluid is charged or withdrawn via the injection device unit 3. This unit may be positioned at the upstream or downstream of the injection device unit 3, so long as the position is adjacent to it.

The extension tube unit 6 includes a tube 60, a connector 61 for being connected to a member for access to a patient such as an injection needle or an catheter, and a connector 62 for being connected to another unit, as the components. The extension tube unit 6 is positioned at the end portion on a patient side and has a function of allowing the infusion line to be held at the hand of the patient easily. The connector 61 may be either of a lure connector or a lock connector. That is, a free lock nut 63 is not an indispensable component. However, it is preferable that the free lock nut 63 is provided so as to be able to lock the connection between the connector 61 and the access member, since thereby it is avoided that the connector 61 is disconnected accidentally from the access member.

General configurations of the units included in the respective unit groups are as described above. However, the combination, size, shape and the like of the components configuring the units may be varied considerably depending on the entire specification required of the infusion line. Therefore, in order to standardize the units, it is necessary to construct a system capable of complying with the various specifications with sufficient flexibility. Therefore, plural kinds of the units with combinations of various kinds of components, sizes, shapes and the like should be provided in each unit group so that the units can properly be selected therefrom according to demands. As a result, the system can be constructed so as to meet broad demands with respect to the entire infusion line.

As described above, when the infusion line is produced according to the method of the present invention, some of the unit groups are selected to determine the combination, and then appropriate units are selected from the respective selected unit groups, so that the combination of the units is determined.

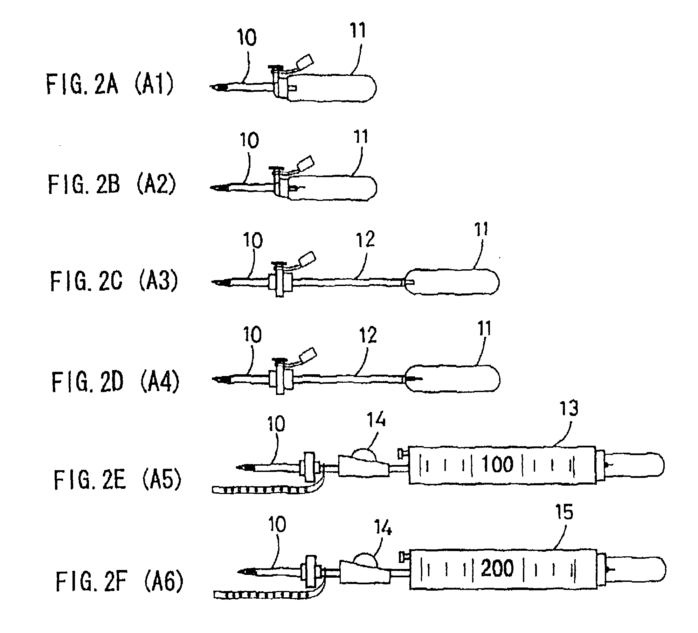

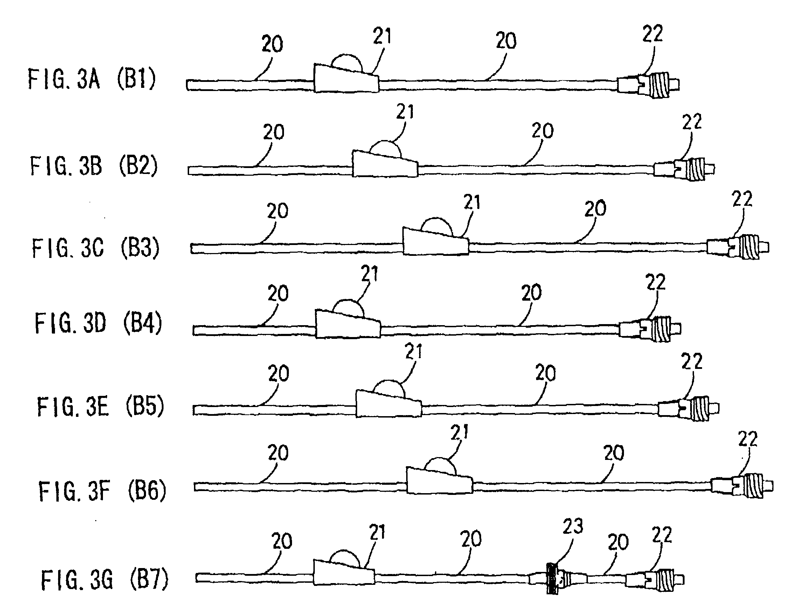

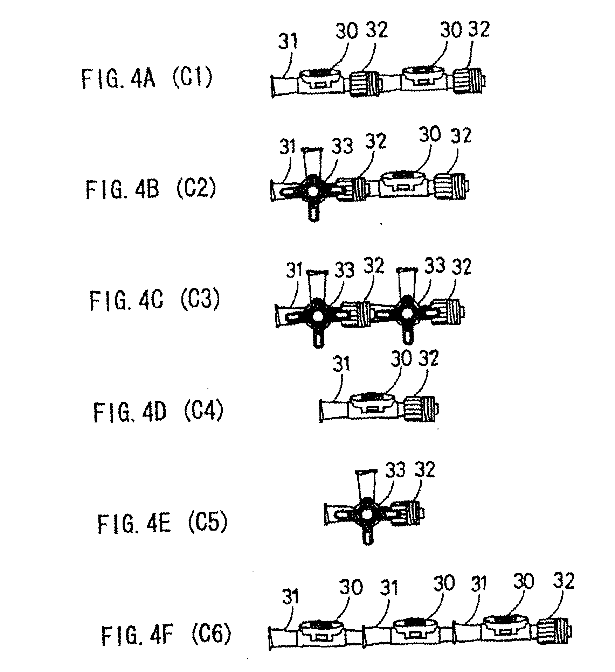

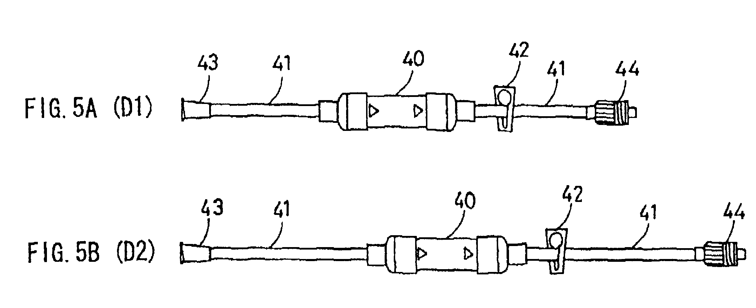

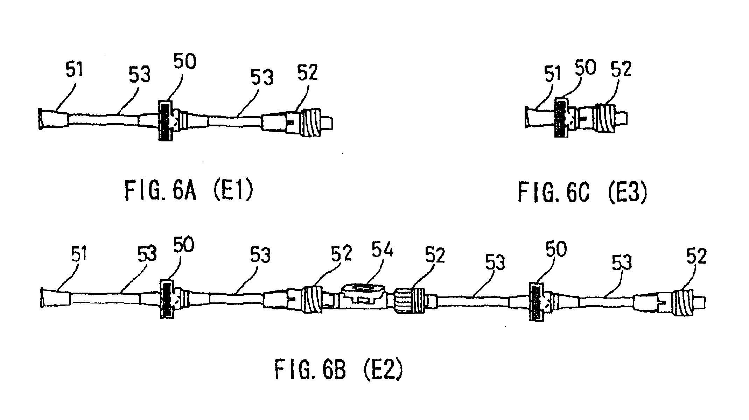

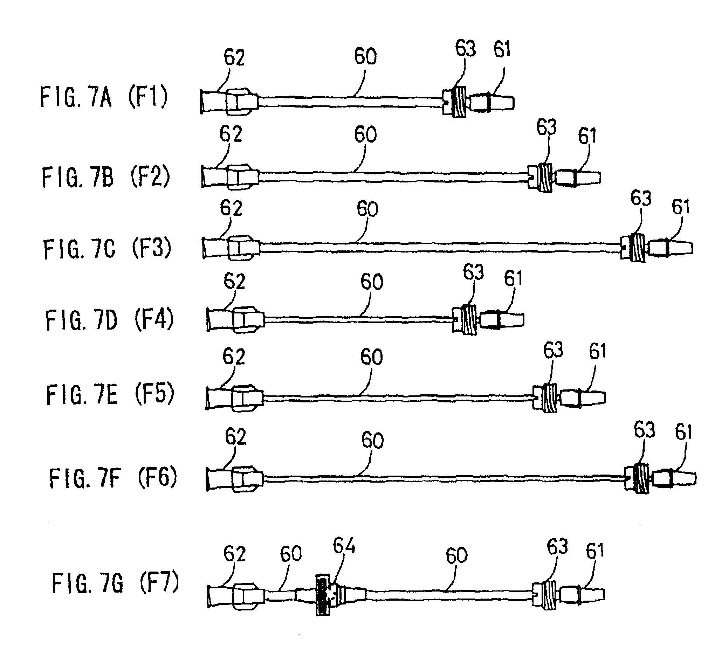

In the following, configuration examples of units will be described with respect to each unit groups.
(A) Units belonging to the spike unit 1
   As shown in FIG. 2A-2F, six types of units denoted as "A1-A6" are provided. A unit A1 is used for injecting normal quantity, and a unit A2 is used for injecting very small quantity. In a unit A3, a tube 12 is inserted between the spike 10 and the drip chamber 11. Aunit A4 is in a shape similar to the unit A3 and is used for injecting very small quandty. In a unit A5, in place of the drip chamber, a quantity measuring barrel or burette 13 is used, and a clamp 14 for controlling a flow rate is inserted between the spike 10 and the burette 13. In a unit A6, a quantity measuring barrel 15 has a larger capacity.
(B) Units belonging to the main tube unit 2
   As shown in FIG. 3A-3G, seven types of units denoted as "B1-B7" are provided. Units B1-B3 are different from one another in full length, while the tubes 20 are same in diameter. Units B4-B6 correspond to the units B1-B3 respectively in full length, while the tubes 20 are different from those of the units B1-B3 in diameter. A unit B7 has a one-way valve 23.
(C) Units belonging to the injection device unit 3
   As shown in FIG. 4A-4F, six types of units denoted as "C1-C6" are provided. In a unit C1, two injection ports 30 are connected to each other. In a unit C2, a three-way stop cock 33 is combined with the injection port 30. In a unit C3, two three-way stop cocks 33 are connected to each other. Aunit C4 includes one injection port 30. Aunit C5 includes one three-way stop cock 33. In a unit C6, three injection ports 30 are connected to one another.
(D) Units belonging to the infusion filter unit 4
   As shown in FIG. 5A-5B, two types of units denoted as "D1" and "D2" are provided. The units D1 and D2 have the structure as mentioned above similar to each other, and differs form each other only in full length.
(E) Units belonging to the one-way valve unit 5
   As shown in FIG. 6A-6C, three types of units denoted as "E1-E3" are provided. A unit E1 is of a basic structure. In a unit E2, two units E1 are connected to each other with an injection port 54 sandwiched. In a unit E3, the one-way valve 50 is connected to the connectors 51 and 52 directly without using tubes.
(F) Units belonging to the extension tube unit 6
   As shown in FIG. 7A-7G, seven types of units denoted as "F1-F7" are provided. Units F1-F3 are different from one another in full length, while tubes 60 are same in diameter. Units F4-F6 correspond to the units F1-F3 respectively in full length, while the tubes 60 are different from those of the units F1-F3 in diameter. A unit F7 has a one-way valve 63.

### [Second Embodiment]

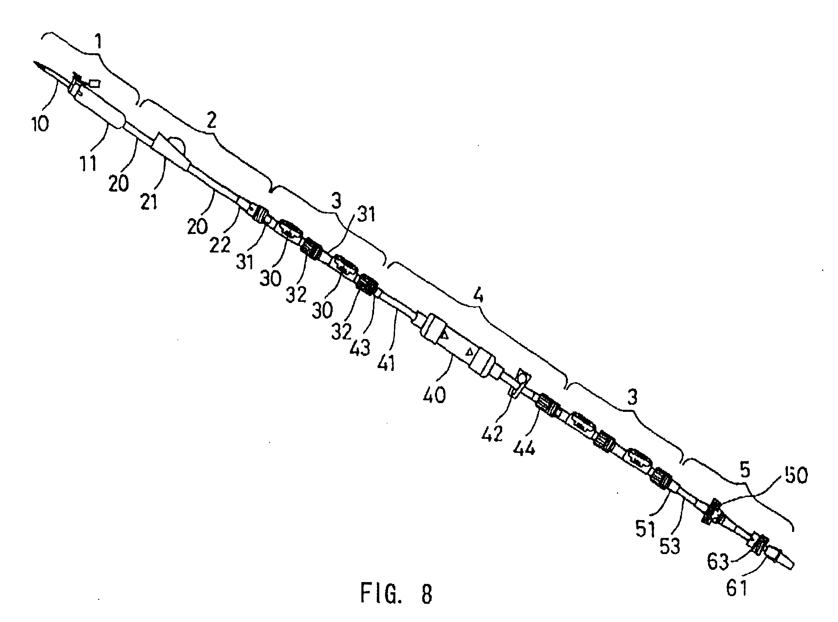

Although the configuration of the infusion line described in the first embodiment includes all of the elements (units) required for a usual practical use. However, it is possible to eliminate the extension tube unit 6 as shown in Fig.8, depending on the purpose of using the infusion line. In such case, a connector forming the end of a unit positioned at the end on a patient side, i.e. the distal side relative to the spike unit 1, is used for connecting with a member for access to a patient. Therefore it is necessary to properly set the configuration of the unit positioned at the end on a patient side. In the configuration as shown in Fig.8, for example, the connector 61 used for the connection with the member for access to a patient is provided at the end of the one-way valve unit 5.

As described above, according to the present invention, it is possible to compose an infusion line by using standardized units, while adopting users' various demands. Furthermore, it is possible to perform simply the operation for composing an infusion line that meets the users' demands by only selecting standardized units. Since each unit has compatibility and is made uniform, reduction in cost due to simplification of the production system can be achieved.

## Claims

1. A method of composing an infusion line, comprising:
classifying components for forming the infusion line into unit groups comprising a spike unit, a main tube unit, an injection device unit, an infusion filter unit, and a one-way valve unit,
providing plural kinds of units for each unit group according to different specifications, each unit being formed of one or more components combined based on a function of the respective unit,
determining a combination of the unit groups by allowing the spike unit and the main tube unit to be indispensable and selecting at least one of the unit groups from the remaining unit groups,
determining an arrangement of the selected unit groups, with the spike unit being positioned at one end thereof,
selecting at least one kind of the units from each of the unit group based on the determined combination, and
combining the selected units according to the determined arrangement to compose the infusion line, with the end on the distal side relative to the spike unit being composed of a connector used for a connection to a member for access to a patient.

2. The method of composing the infusion line according to claim 1, wherein
the unit groups further comprises an extension tube unit, and
when the extension tube unit is selected as one of the unit groups composing the infusion line, the arrangement of the selected unit groups is determined with the extension tube unit being positioned at the end on the distal side relative to the spike unit.

3. The method of composing the infusion line according to claim 2,
wherein the combination of the unit groups are determined with the extension tube unit being included in the indispensable units.

4. The method of composing the infusion line according to claim 1,
wherein
the spike unit has a function of connecting the infusion line to a supply source of a medical fluid via a spike,
the main tube unit is connected to the spike unit and has a function of being used for adjusting a length between the spike unit and another unit positioned on a patient side,
the injection device unit is positioned between the main tube unit and the extension tube unit and has a function of being used for injecting the medical fluid or collecting blood,
the infusion filter unit is positioned between the main tube unit and the extension tube unit and has a function of being used for removing foreign substances,
the one-way valve unit is positioned on the opposite side of the spike unit with respect to the main tube unit and adjacent to the injection device unit and has a function of preventing a back flow of an infusion fluid.

5. The method of composing the infusion line according to claim 2, wherein
the spike unit is positioned at an end portion on a medical fluid side of the infusion line and has a function of connecting the infusion line to a supply source of a medical fluid via a spike,
the main tube unit is connected to the spike unit and has a function of being used for adjusting a length between the spike unit and another unit positioned on a patient side,
the injection device unit is positioned between the main tube unit and the extension tube unit and has a function of being used for injecting the medical fluid or collecting blood,
the infusion filter unit is positioned between the main tube unit and the extension tube unit and has a function of being used for removing foreign substances,
the one-way valve unit is positioned between the main tube unit and the extension tube unit and adjacent to the injection device unit and has a function of preventing a back flow of an infusion fluid, and
the extension tube unit is positioned at an end portion on the patient side and has a function of allowing the infusion line to be held at hand of the patient easily.

6. The method of composing the infusion line according to claim 1,
wherein as the components for forming the units belonging to each unit group,
the spike unit includes a spike and a drip chamber,
the main tube unit includes a tube, a clamp for controlling flow rate, and a connector,
the injection device unit includes a connector and at least one of an injection port and a three-way stop cock,
the infusion filter unit includes an I-V filter, a tube for connection, and a connector, and
the one-way valve unit includes a one-way valve and a connector.

7. The method of composing the infusion line according to claim 2,
wherein as the components for forming the units belonging to each unit group,
the spike unit includes a spike and a drip chamber,
the main tube unit includes a tube, a clamp for controlling flow rate, and a connector,
the injection device unit includes a connector and at least one of an injection port and a three-way stop cock,
the infusion filter unit includes an I-V filter, a tube for connection, and a connector,
the one-way valve unit includes a one-way valve and a connector, and
the extension tube unit includes a tube and a connector.
